(19) Europäisches Patentamt
European Patent Office
Office européen des brevets

(11) **EP 4 749 313 A1**

(12) **EUROPEAN PATENT APPLICATION**

(43) Date of publication:
**27.05.2026 Bulletin 2026/22**

(21) Application number: **24214299.0**

(22) Date of filing: **20.11.2024**

(51) International Patent Classification (IPC):
**G01R 33/12** $^{(2006.01)}$       **A61B 5/0515** $^{(2021.01)}$

(52) Cooperative Patent Classification (CPC):
**G01R 33/1276; A61B 5/0515**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Bruker BioSpin GmbH & Co. KG
76275 Ettlingen (DE)**

(72) Inventors:
• ILBEY, Serhat
 **76337 Waldbronn (DE)**
• FRANKE, Jochen
 **76199 Karlsruhe (DE)**

(74) Representative: **Kohler Schmid Möbus
Patentanwälte
Partnerschaftsgesellschaft mbB
Gropiusplatz 10
70563 Stuttgart (DE)**

(54) **METHOD FOR GENERATING A MULTI-DIMENSIONAL SYSTEM MATRIX FOR MPI-MEASUREMENTS AND METHOD FOR GENERATING AN MPI-IMAGE USING A MULTI-DIMENSIONAL SYSTEM MATRIX**

(57)     The invention concerns a method for generating a multi-dimensional system matrix $A$ for MPI-measurements, the method comprising:
• prior to calibration measurements:
◦ determine
- target positions for a calibration source,
- calibration voxels of a calibration volume
- parameters,
- FFR-offsets r, by which the field free region FFR is to be shifted within the calibration volume,
- acquisition window,

◦ discretize parameters,

• perform calibration measurements, wherein during each calibration measurement, data is acquired for a specific parameter value combination,
• determine individual acquisition system matrices $A_c$, wherein each individual acquisition system matrix $A_c$ combines calibration data derived from different calibration measurements that are assigned to the same parameter value combination but different FFR-offsets r, and
• determine the multi-dimensional system matrix $A$ by combining individual acquisition system matrices $A_c$ of different parameter value combinations.
    The resulting system matrix is not limited to specific MPI-sequences, tracer parameters, MPI scanner parameters, or FFR-geometries.

| determine | -target positions<br>-calibration voxels<br>-parameters<br>-FFR-offsets<br>-acquisition window |
|---|---|

↓

| discretization of parameters |
|---|

↓

| calibration measurements |
|---|

↓

| individual acquisition system matrices $A_C$ |
|---|

↓

| Combination of individual acquisition system matrices |
|---|

↓

**multidimensional system matrix A**

Fig. 1

**Description**

<u>Field of invention</u>

The invention concerns a method for generating a system matrix for MPI-measurements comprising performing calibration measurements and determining the system matrix.

<u>Background of the invention</u>

In magnetic particle imaging (MPI) measurements, a spatially dependent magnetic field with a field-free region (e.g. a field-free point FFP or a field-free line FFL) is applied (selection field). By applying a magnetic shift/focus field, the field-free region is moved through an examination volume (field of view FOV) along a trajectory with the aid of a measurement sequence in a drive-field region. The signal responses of magnetic particles, preferably superparamagnetic iron oxide particles (SPION), are measured as MPI signal data. The measured signal data are displayed as a sum signal of all excited particles. These are measured in the time domain and transformed into the frequency domain by a Fourier transform. A reconstruction is performed to produce the MPI image, e.g. using a system-function-based MPI image reconstruction.

In case of a system-function-based MPI image reconstruction, knowledge of a spatially encoded system response (frequency response), a so-called system function, is used, the latter describing the relationship between the measurement signal (MPI signal data) and, for example, the particle distribution of a certain particle class (mapping the particle concentration on a measured frequency response). As a rule, the system function is available as a "system matrix", i.e. as discretized system function. The system matrix is provided for a system matrix region that comprises the part of the image domain where MPI image data are to be reconstructed. The system matrix provides the basis functions, which describe the signal response of e.g. a calibration sample at various spatial positions. The system matrix is determined (e.g. by calibration measurement, by simulation, by hybrid approaches) independently of the actual object measurement.

For the conventional image reconstruction, a system calibration sequence is performed repeating the MPI sequence to be performed and a source object is placed at each voxel (3D pixel) position within the field of view (FOV). These calibration measurements are then used to construct the system matrix, designated with $A^{M \times N}$, where $M$ represents the number of the frequency components used, and $N$ is the number of voxels. Finally, the image is reconstructed solving the ill-conditioned linear system of equations

$$A\rho = b \qquad\qquad [1]$$

using iterative optimization methods, such as regularized Kaczmarz and FISTA, with the forward model

$$\underset{\rho}{\mathrm{argmin}}\ \|A\rho - b\|_2^2 + \lambda \Phi(\rho) \qquad\qquad [2]$$

where $\rho$ is the image to be reconstructed, $b$ is the measurement vector, $\Phi(\cdot)$ is the regularization operator (for example, $\ell_1$- and $\ell_2$-norm of the image: $\|\rho\|_1$ and $\|\rho\|_2$, respectively), and $\lambda$ is the regularization constant.

A classical system matrix calibration is disclosed in [US2015221103]. This calibration method applies compressed sensing steps with a transformation matrix which sparsifies the image reconstruction matrix, wherein the transformation matrix comprises analytic transformations.

[US20170020407] describes an MPI method to determine calibration and measurement volumes, wherein the calibration volume is larger than the measurement volume and the overall measurement volume is arranged within the calibration volume ("overscanning").

[Kaethner] discloses a method for acquiring MPI data based on the axial elongation of a 2D FFP-trajectory. It is shown that such an elongation can be used as a data acquisition path to significantly increase the acquisition speed, with negligible loss of spatial resolution. However, as a result of the elongation, the 3D measurement volume is not sampled symmetrically and / or homogeneously which may lead to distortions and information loss in the reconstructed images.

[Von Gladiss] discloses the acquisition of a hybrid system matrix with a magnetic particle spectrometer. In contrast to changing the nanoparticle sample position, the nanoparticle sample stays at the same spatial position inside the sample chamber. Different spatial positions are emulated inside the magnetic particle spectrometer by changing the magnetic field that is applied to the particles. When acquiring a hybrid system matrix with a magnetic particle spectrometer the whole sample chamber represents one infinitesimal volume of the FOV in an MPI scanning device. Consequently, the calibration sample size is not constrained to the dimensions of a voxel of targeted image resolution, allowing for the use of a calibration sample as large as the sample chamber itself, provided the magnetic fields are homogeneous throughout, enabling high-SNR calibration measurements. However, the magnetization response of SPIONs can be influenced by environmental factors such as temperature, and viscosity. Additionally, the non-deterministic field drifts and non-linearities present in the MPI scanner etc. may not always be accurately emulated with a magnetic particle spectrometer and need to be compensated (e.g. transfer function compensation, background correction, sensitivity profile correction,...).

[CN115797493] discloses a MPI sampling based on a FFL using an one-dimensional system matrix, where each projection angle (FFL-angle) of the 2D imaging object is acquired separately in a step-wise manner. However, this method does not always work accurately and can only be used for a specific type of sequence.

[US009651637] discloses a method for filtering out interferences from a received signal by applying signal processing techniques like spectral cleaning.

[Halkola] discloses a method for generating a system matrix using an FFP. Instead of moving the nanoparticle sample position by a robot, magnetic focus fields are used for estimating the system function. However, this method is not always applicable using constant magnetic focus fields for calibration measurements using FFL.

Object of the invention

It is an object of the invention to provide a method for generating a system matrix that is not limited to a specific MPI-sequence, tracer parameters, MPI scanner parameters, or FFR-geometries.

Summary of the invention

This object is achieved by a method for generating a system matrix according to claim 1, and a method for generating an MPI-image according to claim 19.

The inventive method is a method for generating a multi-dimensional system matrix for MPI-measurements. Prior to calibration measurements, target positions for a calibration source, calibration voxels of a calibration volume from which calibration data is to be acquired, parameters, and an acquisition window, which is the duration of one calibration measurement to be performed, are determined. The calibration volume comprises a region of interest. The parameters include FFR-velocities of a field-free region of a magnetic selection field, and FFR-offsets, by which the field-free region is to be shifted within the calibration volume. The parameters are discretized prior to the calibration measurements, thereby generating intervals of discretized parameter values for each parameter. Calibration measurements are performed by applying at least one drive field cycle (DF-cycle) of a drive field in each calibration measurement and the calibration data is acquired from the calibration voxels, wherein during each calibration measurement, data is acquired for a specific parameter value combination. All calibration measurements can optionally be transformed into the frequency domain by a Fourier transform. Individual acquisition system matrices $A_c$, are determined, wherein each individual acquisition system matrix $A_c$ combines calibration data derived from different calibration measurements that are assigned to the same parameter value combination but different FFR-offsets. The multi-dimensional system matrix is determined by combining individual acquisition system matrices $A_c$ of different parameter value combinations.

Instead of performing calibration measurements by traversing complete trajectories for multiple source positions (as known from the prior art), the method according to the invention provides that calibration measurements are performed for a plurality of parameter value combinations for each calibration voxel (voxel of the calibration volume from which calibration data is acquired). In order to address different calibration voxels different FFR-offsets are set, wherein an FFR-offset defines the distance between the FFR and origin of region of interest (position of field-free region without application of focus fields) und thus the calibration voxels.

For each FFR-offset, calibration data is obtained for exactly one calibration voxel. I.e. to obtain calibration data from a different calibration voxel, the FFR-offset must be changed. Each calibration measurement is assigned to a specific FFR-offset. In this way, signals for different parameter value combinations are obtained from each calibration voxel. In other words, signals with different FFR-velocities are acquired for each FFR-offset and calibration source position for a duration of an acquisition window. Each parameter value combination comprises values of determined parameters. Parameters used for parameter combinations comprise sequence parameters, i.e. parameters which are relevant for MPI-sequences used for the MPI-measurements (e.g. FFR-velocities), and may comprise further parameters, which are relevant e.g. for sample to be imaged (e.g. temperature, viscosity, type, size, coating, shape...) and/or the excitation used for imaging (e.g. drive field frequency, drive field amplitude) and/or selection field used for imaging (e.g. gradient and shape of the field-free region such as field-free line and field-free point), receiver coils used / inserted and receiver coil orientations.

According to the invention, individual acquisition system matrices are generated, each individual acquisition system matrix comprises calibration data for a specific parameter value combination. The combination of these individual acquisition system matrices is done by concatenating the individual acquisition system matrices and results in the multidimensional system matrix. The dimension of the multidimensional system matrix according to the invention depends on the number of parameters and comprises short data sections associated with each parameter and spatial positions, in contrast to conventional system matrices consisting of repeated full MPI trajectory measurements for different spatial positions.

The calibration volume comprises a region of interest and eventually an overscanning region. The region of interest is the region to be imaged; the overscanning region is the area of the calibration volume that is not reached by the FFR trajectory, but for which calibration measurements are acquired and ultimately reconstructed.

Prior to the calibration measurements, the size of the calibration volume and number of voxels in each direction of the calibration volume and thus the resolution of the calibration measurements are determined. The size and orientation of the calibration voxels may be different depending on FFR-shapes, FFL-angles, the region of interest and the overscanning region. In case the FFR is a FFL, for example, the calibration volume can also be defined in sinogram domain. The FFR-offset is always orthogonal to the FFL-orientation. Thus, the calibration voxels' orientation rotate with FFL-angle.

According to the invention the parameters are discretized, i.e. a continuous parameter is transformed into a discrete set of distinct parameter values. This enables calibration measurements with a multitude of parameter value combinations over the whole parameter space.

According to the invention, individual acquisition system matrices are determined. Each individual acquisition system matrix comprises data from different offsets and may also comprise data from different source positions.

At least one of the positions of the calibration source is preferably chosen in the center of the region of interest. The positioning of the calibration source within the region of interest can be realized mechanically by using a precise robot or a moving animal/sample table and/or by using magnetic fields (shift/focus fields) for shifting the field of view relative to the calibration source.

Since the acquired calibration data originates from MPI hardware components such as power amplifiers, filters, and cabling etc., interferences with MPI receive coils can occur. In order to remove such interferences, signal processing techniques like spectral cleaning can be applied to all calibration data and imaging data.

A DF-cycle is one period of the HF-drive field. In a special variant, during a drive field cycle, the focus fields are kept constant (step-wise imaging). Alternatively, dynamic imaging is possible, where the focus field changes during a drive field cycle. The change of the focus field per duration of one drive field cycle is defined as FFR velocity, i.e. the speed the FFR moves in space in the direction of the offset field or mechanical movement. The drive field direction is preferably perpendicular to the direction of the focus field. In a special embodiment, there can be multiple drive field and focus field directions.

In a preferred variant, for setting a specific FFR-offset, the field-free region is shifted in focus-field directions by applying according focus fields and/or the field-free region is shifted in drive field direction by mechanically shifting the calibration source.

It is preferred that for each FFR-offset, calibration data is acquired from each calibration voxel for different parameter value combinations and/or for each parameter value combination, calibration data is acquired from each calibration voxel.

In a special variant, during each calibration measurement, calibration data is acquired from one specific calibration voxel only. Thus, acquisition duration of the multidimensional system matrix is minimized.

In a specific variant, the following steps are performed:

i. the calibration source is positioned within the region of interest at a first target position,

ii. calibration measurements with the calibration source being positioned at a first target position, wherein for each calibration measurement the field-free region is offset to a specific calibration voxel, and wherein calibration measurements are performed for all predefined parameter value combinations until all FFR-offsets have been set,

iii. the calibration source is positioned within the region of interest at a further target position that is shifted relative to the previous target position within the region of interest in at least one direction, preferably along a drive field direction, and

iv. calibration measurements with the calibration source being positioned at the further target position,

v. steps iii - iv are repeated until measurements for all target positions of the calibration source are performed.

In particular, in a more specific variant, in step ii, for each FFR-offset, predefined parameter value combinations are set and the FFR-offset is changed after calibration measurements have been performed for all predefined parameter value combinations. I.e, first the parameter combination and then the FFR-offset are varied. This variant is preferred, if transition time (e.g. milliseconds) to increment FFR-offset is larger compared to incrementing the other parameters.

Preferably, one FFR-velocity is detected first in the shift/focus field directions, followed immediately by the reverse FFR-velocity (sign change). This order of acquisition is particularly advantageous for the practical implementation of general system matrix detection since the hardware can thus better support the transition to the next FFR-velocity.

In another specific variant, the following steps are performed:

i. the calibration source is positioned within the region of interest at a first target position,

ii. calibration measurements with the calibration source being positioned at a first target position, wherein for each calibration measurement, a predefined parameter value combination is set, and wherein calibration measurements are performed until all predefined parameter value combinations have been set,

iii. the calibration source is positioned within the region of interest at a further target position that is shifted relative to the previous target position within the region of interest in at least one direction, preferably along a drive field direction, and

iv. calibration measurements with the calibration source being positioned at the further target position,

v. steps iii) - iv) are repeated until measurements for all target positions of the calibration source are performed.

In particular, in a more specific variant, in step ii, for each parameter value combination, predefined FFR-offsets are set and the parameter value combination is changed after calibration measurements have been performed for all predefined FFR-offsets. I.e, first the FFR-offset and then the parameter combinations are varied. This variant is preferred if transition time (e.g. milliseconds) to increment FFR-offset is shorter compared to incrementing the other parameters.

In order to remove background noise, background measurements can be performed, preferably prior to positioning the calibration source at the target positions in step i and step iii, without the calibration source being in the calibration volume and/or with the calibration source being in a magnetically saturated state.

Preferably, the determined parameters comprise at least one of the following: particle type of used tracer of the calibration source, temperature of the calibration source, viscosity of the calibration source, velocity of the calibration source per acquisition window, DF frequency, DF amplitude, magnetic field gradient of an MPI system used for the MPI measurements, duration of the acquisition window, position of optional hardware inserts, shape of the field-free region, receiver coils used, receiver coil orientations. Although the consideration of additional parameters increases the total acquisition time and memory requirements, it enables the use of the same multidimensional system matrix for any arbitrary trajectory in a multitude of framework conditions. In this way, the MPI system user does not need to do very tedious calibration measurements repeatedly for each further parameter.

When imaging large objects, it is advantageous if the object being imaged is moved at a preferable constant speed during the MPI sequence. This method enables more efficient data acquisition across the entire 3D FOV, thereby minimizing the total measurement time. For the calibration measurements, the same physical shift can be mimicked by moving the calibration source during a calibration measurement at a given speed. After each calibration measurement, source object returns to its initial position. This speed variable must also be discretized.

The discretization can be performed such that the parameter values are uniformly or non-uniformly spaced. Non-uniformly spaced parameter values can be preferable if, for example, a particular range of parameter values are statistically expected to be used more frequently and can be spaced in finer increments.

In a highly preferred variant, data acquisition for the calibration measurements are carried out for the same calibration voxel with all possible parameter value combinations, i.e. all permutations of parameter values are applied during signal acquisition of each calibration voxel. The resulting multi-dimensional system matrix is a **generic system matrix,** which is acquired independently of a particular MPI-sequence to be performed and which can be used for reconstruction of MPI-images from data acquired from MPI-measurements using any arbitrary MPI-sequence.

Each calibration measurement is assigned to a specific calibration voxel and thus to a specific FFR-offset. I.e. for each calibration voxel, a multitude of calibration measurements (each calibration measurement with one specific parameter value combination) are performed, wherein the number of calibration measurements per calibration voxel is the number of parameter value combinations.

In an alternative variant, parameter value combinations are determined from a predetermined MPI-sequence and calibration measurements with all parameter value combinations specific for the MPI-sequence are performed.

The resulting multi-dimensional system matrix is a **trajectory-specific system matrix,** which can be used for a specific MPI-sequence to be performed. The predetermined MPI-sequence is the MPI-sequence (trajectory of the FFR) to be performed during a future MPI-measurement. Prior to the calibration measurements, the combinations of parameter values that occur in the predetermined MPI-sequence are determined (trajectory specific parameter value combinations) and all trajectory specific parameter value combinations are applied during signal acquisition of each calibration voxel. I.e. calibration measurements for all trajectory-specific parameter values combinations are performed for each FFR-offset.

Each calibration measurement is assigned to a specific calibration voxel and thus to a specific FFR-offset. I.e. for each calibration voxel, a multitude of calibration measurements (each calibration measurement with one parameter value combination) is performed, wherein the number of calibration measurements per calibration voxel is the number of trajectory specific parameter value combinations.

During each calibration measurement, a positive integer multiple of DF-cycles can be applied, preferably a single digit number of DF-cycles, most preferably one DF-cycle. The duration of the calibration measurements (acquisition window) should be chosen as short as possible in order to ensure a feasible total calibration duration, but long enough for the particles in the calibration source to generate a signal with sufficient Signal-to-Noise Ratio (SNR).

In a special variant, the field-free region is a field-free line, and the parameters comprise FFL-angle, FFL-angular-velocity and FFL-translational-velocity. Using a field-free line is advantageous in terms of sensitivity and SNR because the SPIONs within the entire field-free line contribute to the signal. Alternatively, the field-free region can be a field-free point, and the parameters comprise FFP-velocities in any spatial direction. When a field-free point is used, a very small sub-section of a region of interest can be imaged, enabling ultra-fast imaging. However, in principle, the shape of the FFR is not limited to FFP or FFL.

In a highly preferred variant, in order to move the calibration source between the target positions, the calibration source is moved relative to the region of interest exclusively in the drive field direction. I.e. calibration measurements are performed with a fixed source position regarding the xy plane with a plurality of parameter value combinations for each calibration voxel. The position of calibration source is preferably chosen in the origin of the region of interest.

In order to reduce the time required for the calibration measurements, it is proposed that the calibration volume consists of n voxels, wherein the number m of calibration voxels is smaller than the number n of voxels of the calibration volume, and the calibration data for voxels not being calibration voxels is interpolated and/or extrapolated from the already acquired calibration data of the calibration voxels. I.e. one can do undersampling and skip some voxels of the calibration volume. In other words, the FFR is shifted to only some of the voxels of the calibration volume.

In a special variant, only one voxel of a group of voxels of the calibration volume, which are geometrically symmetrical to each other, is a calibration voxel. Due to geometrical symmetries, calibration data of the voxels of the above-mentioned group of voxels that are not a calibration voxel can be generated, e.g. by phase shifting, amplitude scaling, and/or flipping.

Likewise, undersampling can be done with regard to the parameters.

The invention also concerns a method for generating an MPI-image from MPI-data which have been acquired by performing an MPI sequence, the method using a multi-dimensional system matrix generated according to a method described before.

According to the invention, the MPI-sequence is divided into trajectory-sections, whereby the duration of each trajectory-section corresponds to the duration of the acquisition window, each trajectory-section being associated with an actual parameter value combination and FFR-offset, wherein during each trajectory-section a data-subset of the MPI-data is acquired. For each trajectory-section, the best matching individual acquisition system matrix $A_c$ is picked from the multi-dimensional system matrix A, wherein the best matching individual acquisition system matrix $A_c$ is the one with the parameter value combination that best matches the actual parameter value combination of the respective trajectory-section, where $c^{+\mathbb{Z}} \in [1, C]$ is the trajectory-section number and C is the total number of trajectory-sections. An image-resolution is determined of the MPI-image to be generated, wherein the image-resolution comprises voxel sizes and FOV size. The FOV (field of view) corresponds to the calibration volume (including the overscanning region). After an optimization algorithm is chosen, the MPI-image is reconstructed by applying the chosen optimization algorithm to the MPI-data using the picked best matching individual acquisition system matrices.

The overscanning region is optionally clipped out from the resulting image.

The inventive method for generating an MPI-image is based on fragmenting the trajectory and assigning individual acquisition system matrices (each comprising calibration data for a specific parameter value combination) of the multidimensional system matrix to the respective trajectory sections.

In a first variant **(single section based FFP-variant),** the field-free region is a field-free point, and the MPI-image is reconstructed by performing the following ste ps :

- for each data-subset, perform iterative reconstruction, wherein each reconstruction results in an individual image $x_c$,

- weight and sum up all individual image $x_c$, resulting in the MPI-image.

Reconstruction in which for each trajectory-section, iterative reconstruction is performed, wherein each reconstruction results in an individual image, is referred to as a "single section based reconstruction method", where a voxel-wise summation operation is performed. As the resulting individual images $x_c$ are centered to the FFR-offset of their associated data-subset, it is preferred to aligned them to the center of the FOV by shifting, if/when needed. Alternatively, prior to iterative reconstruction, all individual acquisition system matrices $A_c$ can be shifted by the associated FFR-offset of trajectory section c along their offset dimensions. Prior to the voxel-wise summation operation, the reconstructed individual images can be filtered, preferably by a spatial filter which scales down values at voxel positions further away from the position of the field-free point, to obtain sharper images. Further, weighting of the data-subsets according to a sampling density can be performed for each voxel position from which the according MPI-data-subset has been acquired.

Since the field-free region is a field-free point (FFP), the reconstructed data is linearly related to the mass of SPIONs.

In a second variant **(one-shot FFP-variant),** the field-free region is also a field-free point. However, in this variant, the MPI-image is reconstructed by performing the following steps:

- concatenate all data-subsets, resulting in a measurement vector $b_{traj}$

- concatenate all individual acquisition system matrices $A_c$ with the same order used for forming $b_{traj}$, resulting in a calibration matrix $A_{traj}$,

- to obtain the MPI-image: perform iterative reconstruction.

In contrast to the single section based variant, the individual acquisition system matrices as well as the trajectory-sections are concatenated prior to the iterative reconstruction in the one-shot FFP-variant. Only one iterative reconstruction has to be carried out ("one-shot reconstruction method").

In a highly preferred variant, the individual acquisition system matrices A_c, are shifted by the associated FFR-offset of trajectory section c along their offset dimensions prior to concatenation.

In a third, fourth and fifth variant, the field-free region is a field-free line. In this case, prior to reconstruction the following steps are performed:

- for the given image resolution, determine a corresponding sino-resolution of a sinogram to be generated, the sino-resolution comprising discretized FFL-angles and FFL-offsets,

- determine positions of the data-subsets within the sinogram.

Forming a sinogram (determination of sino-resolution and determine positions of the trajectory-sections within the sinogram) is relevant if a field-free line is used as a field-free region, because each reconstructed individual image, $x_c$, represents a projection of the 3D image at the given FFL-angle and FFL-offset during trajectory-section c. Each column of a sinogram corresponds to projections of the MPI-image at different projection angles. A sinogram is defined with discrete angles.

In the third variant **(single section based FFL-variant),** the MPI-image is reconstructed by performing the following steps:

- for each data-subset, perform iterative reconstruction, wherein each reconstruction results in an individual image $x_c$,

- weight and sum up all individual image $x_c$, resulting in the sinogram, and

- apply an inverse Radon transform to the sinogram resulting in the MPI-image.

As the first variant, this variant uses a single section based reconstruction method. As resulting individual images $x_c$ are centered to the FFR-offset of their associated data-subset, it is preferred to aligned them to the center of the FOV by shifting, if/when needed. Alternatively, prior to iterative reconstruction, all individual acquisition system matrices $A_c$ can be shifted by the associated FFR-offset of trajectory section c along their offset dimensions. Prior to the summation operation, the reconstructed individual images can be filtered, preferably by a spatial filter which scales down values at voxel positions further away from the position of the field-free line, to obtain sharper images. Further, weighting of the MPI-data-subsets according to a sampling density can be performed for each voxel position from which the according MPI-data-subset has been acquired.

The fourth variant **(column based FFL-variant)** comprises performing the following steps:

- assign each FFL-angle of the trajectory-sections to one column of the sinogram,

- for each sinogram column: select trajectory-sections, whose FFL-angle was assigned to the respective sinogram column,

- concatenate all data-subsets acquired during the selected trajectory-sections, resulting in a measurement vector $b_\theta$,

- concatenate all individual acquisition system matrices $A_c$ with the same order used for forming measurement vector $b_\theta$, resulting in a calibration matrix $A_\theta$,

- perform iterative reconstruction for each discretized FFL-angle, resulting in columns $x_\theta$, of a reconstructed final sinogram,

- to obtain the MPI-image: apply an inverse Radon transform to the reconstructed final sinogram.

In this variant, the trajectory-sections are grouped according to their FFL-angles. Trajectory sections with the same FFL-angle are not necessarily acquired at consecutive time intervals. Such reconstruction, in which sinogram columns are treated separately, is referred to as an "column-based reconstruction method".

The order in which the individual acquisition system matrices are concatenated correspond to the order in which the MPI-data-subsets acquired during the selected trajectory-sections are concatenated. Prior to concatenation of individual acquisition system matrices $A_c$, they must be shifted by the associated FFR-offset of trajectory section c along their offset dimensions.

The fifth variant **(one-shot FFL-variant)** comprises performing the following steps:

- concatenate data-subsets acquired during all trajectory-sections, resulting in a measurement vector $b_{traj}$,
- concatenate all individual acquisition system matrices $A_c$ with the same order used for forming $b_{traj}$, resulting in a calibration matrix $A_{traj}$,
- perform iterative reconstruction,
- to obtain the MPI-image: apply an inverse Radon transform.

As in the second variant, only one iterative reconstruction has to be carried out (one-shot reconstruction method). Prior to concatenation of individual acquisition system matrices $A_c$, they must be shifted by the associated FFR-offset of trajectory section c along their offset dimensions.

For the single section based variants (first and third variant), it is preferred that the optimization algorithm is based on a forward model in particular the following:

$$\underset{x_c}{\mathrm{argmin}}\ \|A_c x_c - b_c\|_2^2 + \lambda_c \Phi(x_c)$$

where

$A_c$ = individual acquisition matrix,
$x_c$ = individual image,
$b_c$ = acquired MPI-data-subset during trajectory-section c,
$\lambda_c$ = regularization constant for trajectory-section c,
$\Phi(\cdot)$ = regularization operator.

For the column based variant (fourth variant), it is preferred that the optimization algorithm is based on a forward model, in particular the following:

$$\underset{x_{col}}{\mathrm{argmin}}\ \|A_\theta x_\theta - b_\theta\|_2^2 + \lambda_\theta \Phi(x_{col})$$

where

$A_\theta$ = calibration matrix,
$x_\theta$ = column of the sinogram for FFL angle $\theta$,
$b_\theta$ = measurement vector,
$\lambda_\theta$ = regularization constant for FFL-angle $\theta$,
$\Phi(\cdot)$ = regularization operator.

For the single one-shot variants (second and fifth variant), it is preferred that the optimization algorithm is based on a forward model, in particular the following:

$$\underset{x_{traj}}{\operatorname{argmin}} \left\| A_{traj} x_{traj} - b_{traj} \right\|_2^2 + \lambda_{traj} \Phi\left(x_{traj}\right)$$

where

$A_{traj}$ = calibration matrix,
$x_{traj}$ = sinogram,
$b_{traj}$ = measurement vector,
$\lambda_{traj}$ = regularization constant,
$\Phi(\cdot)$ = regularization operator.

Further advantages of the invention can be derived from the description and the drawings. Also, the above-mentioned and the still further described features can be used according to the invention individually or in any combination. The embodiments shown and described are not to be understood as a conclusive list, but rather have an exemplary character for the description of the invention.

Brief description of the drawings

Fig. 1        shows the basic method steps of the inventive method for generating a multi-dimensional system matrix.

Fig. 2        shows the steps of a first variant of calibration data acquisition with varying parameter value combinations for each FFR-offset.

Fig. 3        shows the steps of a first variant of calibration data acquisition with varying FFR-offsets for each parameter value combination.

Fig. 4        shows the basic method steps of the inventive method for generating an MPI image.

Fig. 5        shows a single section based FFP-variant of the method shown in Fig. 4 (first variant)

Fig. 6        shows a single section based FFL-variant of the method shown in Fig. 4 (third variant)

Fig. 7        shows a one-shot FFP-variant of the method shown in Fig. 4 (second variant)

Fig. 8        shows a one-shot FFL-variant of the method shown in Fig. 4 (fifth variant)

Fig. 9        shows a column based FFL-variant of the method shown in Fig. 4 (fourth variant)

Fig. 10      shows a calibration volume with overscanning region with a superimposed trajectory T.

Fig. 11a-d   show FFR-offset-positions for 1D source movement.

Fig. 12      shows a region of interest and an FFL with its FFL-parameters.

Fig. 13      shows a sinogram with a superimposed trajectory T.

Detailed description of the drawings

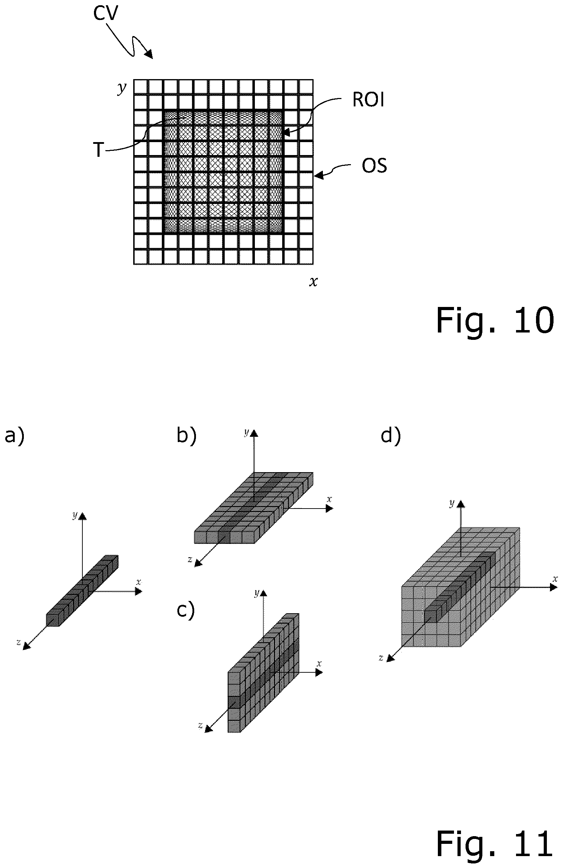

**Fig. 10** shows a calibration volume **CV** (image domain x, y) with a region of interest **ROI,** from which an MPI-image is to be generated, and an overscanning region **OS** (2D-illustration). Each **voxel** of the calibration volume CV, from which calibration data is acquired, is called "calibration voxel". In a 3D calibration volume CV, a third dimension z is added.
Within the region of interest ROI a Lissajous trajectory T is indicated as example along which a field free region FFR is moved during applying an MPI-sequence. According to the prior art, this trajectory T is also run during calibration measurements. The trajectory T is repeated for any source position within the region of interest ROI. The data from each calibration voxel gives information for a specific parameter value combination only, namely that which is present at the calibration voxel, when the trajectory T is run.

In most applications, the field-free region is a field-free point FFP or a field-free line FFL. During tracing the trajectory T the FFP-velocities and the FFL-angle, FFL-angular-velocity, FFL-translational-velocity respectively change.

According to the invention, a method is suggested for generating a multidimensional system matrix, which is not limited to be used with a specific trajectory but can be used for a multitude of trajectories T. In contrast to the methods known from the prior art, the inventive method does not comprise running a specific trajectory repeatedly for different target positions of the source, but to shift the FFR to predefined calibration voxels and set a multitude of parameter value combinations, wherein a calibration data is acquired for all FFR-offsets and all set parameter value combinations. With the inventive method, it is possible to move the source only in one direction within a 3D region of interest ROI (e.g. in z-direction, while staying at x=0, y=0) or to not move the source within a 2D region of interest ROI (e.g. at x=0, y=0 within the xy-plane). In order to nevertheless ensure that signals originating from off-centered positions (x≠0 | y≠0) are also taken into account in the generation of the system matrix, which is essential for accurate image reconstruction, calibration measurements with corresponding FFR-offsets are repeated for each off-centered position in accordance with the invention. **Fig. 11a** shows positions of the source object (dark colored) used for calibration with 1D movement of the source, here in the z-direction as example (e.g. for 1D excitation). **Fig. 11b** and **Fig. 11c** show offset-positions of the FFR (light colored) for the case that an FFL with extension in y-direction for FFL-angle is 0° (Fig. 11b) and x-direction for FFL-angle is 90° (Fig. 11c) is used. The FFL-offset is orthogonal to the extension of the FFL, so the direction of the FFL-offsets rotates with the rotating FFL, thus, the orientation of the calibration voxels changes with FFL angle. Fig. 11d shows offset-positions of the FFR (light colored) for the case that an FFP is used.

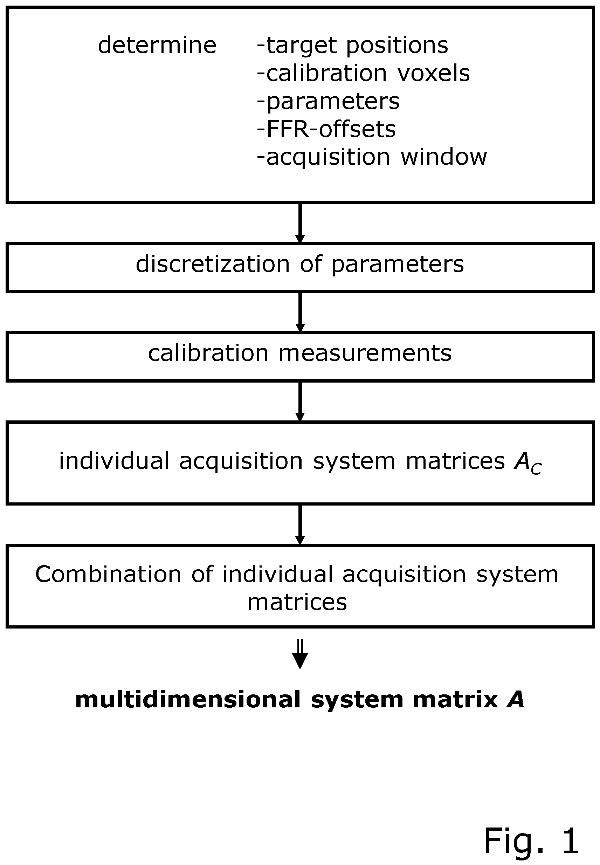

The basic method steps of this method are shown in **Fig. 1.** First, relevant items required to perform the method are determined: positions to which the source is to be moved within the region of interest ROI (target positions), calibration voxels from which signals are to be acquired (calibration voxels), parameters to be varied in order to get information for different parameter value combinations, positions to which the FFR is to be shifted, wherein the FFR is preferable not only shifted within the region of interest ROI but also to the overscanning region OS and acquisition window during which a calibration measurement is to be performed.

In contrast to running complete trajectories, the parameters are discretized prior to perform the calibration measurements. After the calibration measurements, individual acquisition system matrices (with dimensions frequency (or time) and FFR-offset) are determined. By combining the individual system matrices $A_c$ the multidimensional system matrix $A$ is acquired. For example, for the multidimensional system matrix $A$ with dimensions frequency, FFL-offset, calibration source positions $z$ along the z-direction at the center of the ROI ($x = y = 0$), FFL velocity, and FFL angle, can be represented in terms of is individual acquisition system matrices $A_c$ as following:

$$A(f, r, z, v = v_c, \theta = \theta_c) = A_c(f, r, z)$$

where the individual system matrices $A_c$ is acquired for FFL velocity $v_c$ and FFL angle $\theta_c$.

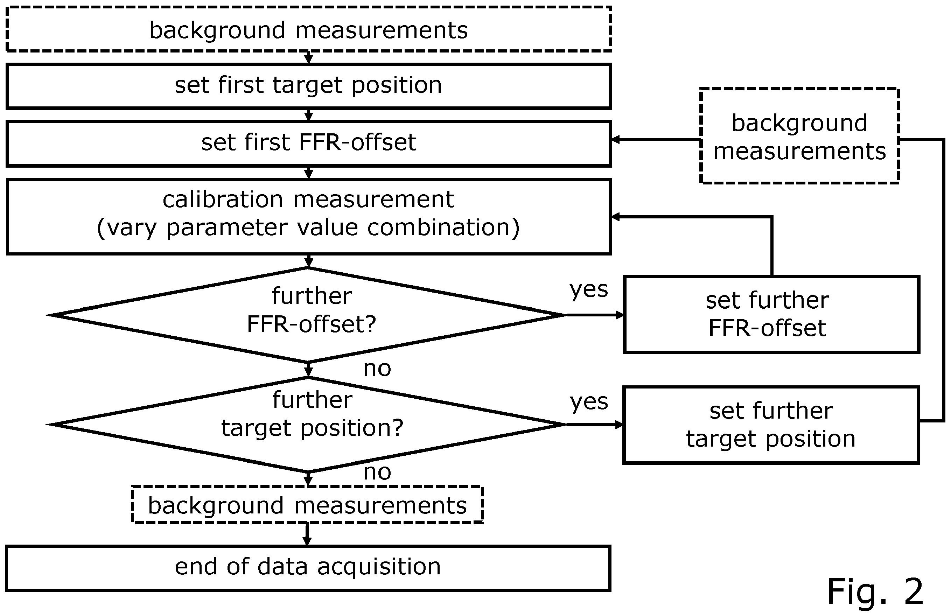

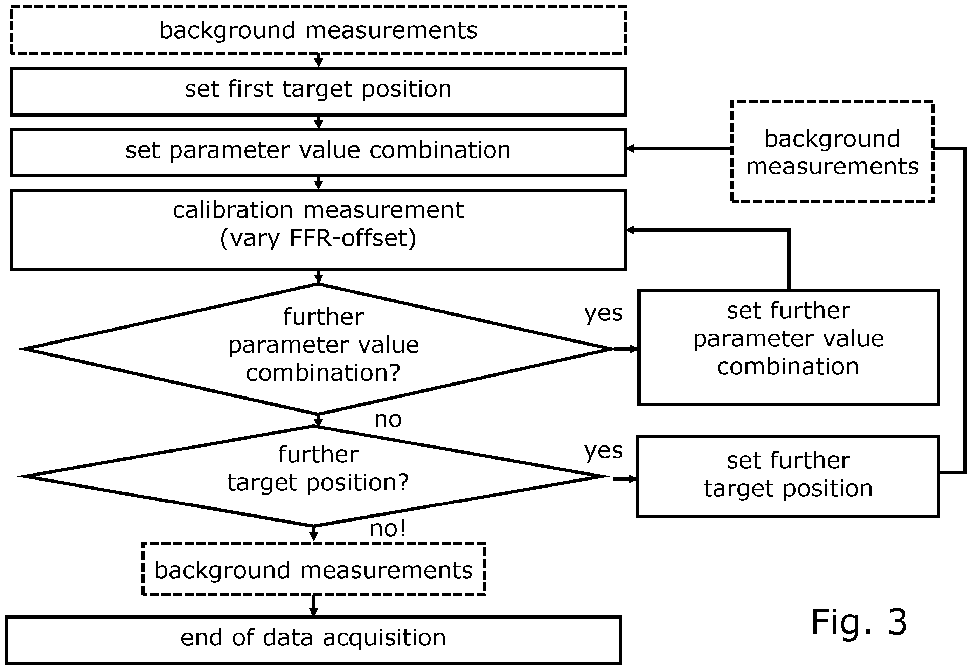

**Fig. 2** and **Fig. 3** show two preferred variants how to perform the calibration measurements. First, the source is positioned in a first target position. Preferably prior to positioning the source, background measurements can be carried out.

The variant shown in **Fig. 2** provides that the field-free region is shifted by a first FFR-offset (first FFR-offset is set) and that for this FFR-offset parameter value combination is varied, wherein for each parameter value combination at least one calibration measurement is performed. This is repeated for each FFR-offset. Then the source is positioned at a further target position and the procedure is repeated for all target positions.

The variant shown in **Fig. 3** provides that a first parameter value combination is set and that for this parameter value combination the FFR-offset is varied, wherein for FFR-offset at least one calibration measurement is performed. This is repeated for each parameter value combination. Then the source is positioned at a further target position and the procedure is repeated for all target positions.

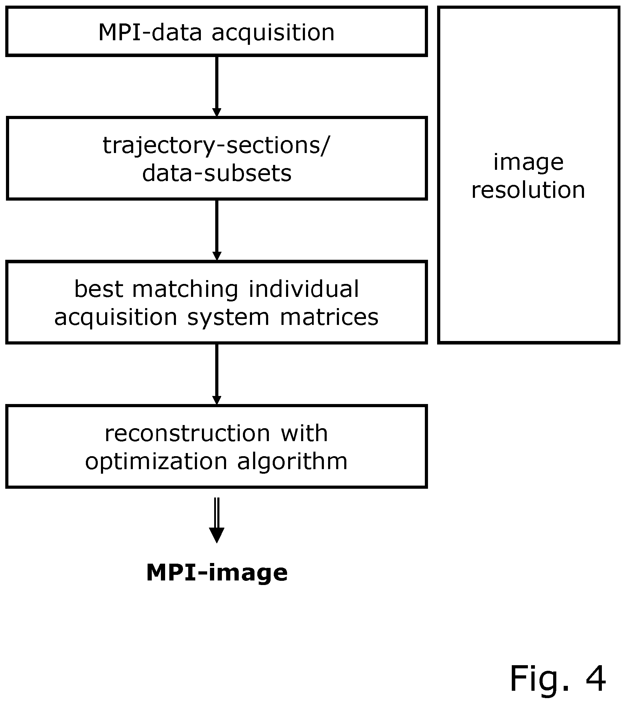

**Fig. 4** shows the basic method steps of the inventive method for generating an MPI image from MPI-data s(t) acquired by a data acquisition. The inventive method uses individual acquisition system matrices of the afore described multidimensional system matrix $A$. The inventive method is based on dividing the trajectory used during MPI-data acquisition as well as the MPI-data acquired during the same MPI-data acquisition into "snippets", i.e. trajectory sections c and data-subsets related to the trajectory-sections c respectively. Each trajectory section has the duration of the acquisition window determined during generation of the multidimensional system matrix. For each trajectory-section the best matching individual system matrix from the multidimensional system matrix is selected. The selected best matching individual acquisition system matrices are used for reconstruction resulting in the MPI-image. For reconstruction an optimization algorithm is applied, e.g. in the form of:

$$\underset{x_{reco}}{\operatorname{argmin}} \; \|A_{reco} x_{reco} - b_{reco}\|_2^2 + \lambda \Phi(x_{reco})$$

where

$A_{reco}$ = calibration matrix,
$x_{reco}$= result of the reconstruction,
$b_{reco}$ = Data to be reconstructed from (measurement vector),
$\lambda$ = regularization constant,
$\Phi(\cdot)$ = regularization operator.

The individual acquisition system matrices are either used directly as a calibration matrix or to generate the calibration matrix. When multiple receiver coils (for example) at different orientations are used, $A_{reco}$ and $b_{reco}$ from each channel can be concatenated prior to iterative reconstruction. I.e. $A_{reco}$ and $b_{reco}$ will comprise calibration matrices and measurement vectors of each channel concatenated, respectively.

In case of using an FFL, the reconstruction cannot be performed directly to the image domain (x, y, z), but to a sinogram domain ($\theta$, r, z):

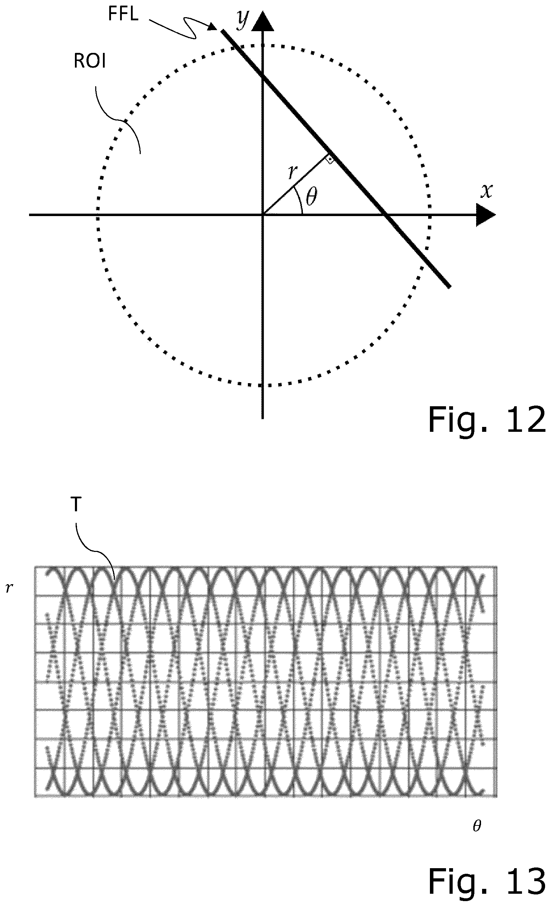

**Fig. 12** shows a field free line **FFL** in the region of interest ROI with an FFL-angle $\theta$ (angle between the FFL-orientation and the x-axis) and an FFL-offset **r** (distance of the FFL to the origin). Translation and rotation of the FFL take place in x- and y-directions, which is defined by magnetic shift/focus fields. A 3D field of view FOV can be imaged by rotating and shifting the FFL in the x- and y-directions between (step-wise) or during (dynamically) drive field excitation cycles. In other words, by modulating the shift or focus fields to cover each (x,y) position, the field of view FOV can be traversed, for example, using a step-wise meander or dynamic sinusoidal trajectory as shown in **Fig. 13** for the dynamic case. A translation (offset) of the FFL corresponds to moving vertically in the sinogram, whereas the rotation corresponds to moving horizontally. Moving in the z-direction (e.g., by DF excitation or focus/shift fields) corresponds to moving orthogonal to the 2D sinogram.

The fields in 3D generated with the exemplary dynamic sinusoidal trajectory with 1D excitation in the z-direction can be defined with the following equations:

$$\boldsymbol{H}(\theta) = G \begin{bmatrix} \frac{1}{2} + \frac{1}{2}\cos 2\theta & \frac{1}{2}\sin 2\theta & 0 \\ \frac{1}{2}\sin 2\theta & \frac{1}{2} - \frac{1}{2}\cos 2\theta & 0 \\ 0 & 0 & 1 \end{bmatrix} \begin{bmatrix} x \\ y \\ z \end{bmatrix}, \quad \theta = 2\pi f_{rot} t$$

$$\boldsymbol{H_{DF}}(t) = \begin{bmatrix} 0 \\ 0 \\ A_{DF}\sin(2\pi f_{DF} t) \end{bmatrix}$$

$$\boldsymbol{H_{FF}}(t) = \begin{bmatrix} H_{FF}\sin(2\pi f_{tra} t)\cos(2\pi f_{rot} t) \\ H_{FF}\sin(2\pi f_{tra} t)\sin(2\pi f_{rot} t) \\ 0 \end{bmatrix}, \quad H_{FF} = G\frac{FOV_{xy}}{2},$$

where G is the gradient of the magnetic fields, $A_{DF}$ and $f_{DF}$ are drive field amplitude and frequency, respectively, and $f_{rot}$ and $f_{tra}$ are the rotational and translational frequencies of the trajectory, respectively.

Accordingly, prior to reconstruction, the resolution of the sinogram (sino-resolution) and the positions of the data-subsets within the sinogram have to be determined. The sino-resolution is determined as follows: The x-axis of the sinogram corresponds to the angle of the FFL. The y-axis corresponds to the offset of the FFL. Assuming that the image has N $\times$ N voxels, from -FOV/2 to FOV/2 in both x- and y-directions, angles of the sinogram (x-axis) go from 0 to 180°, with 180°/N/(pi/2) steps according to the Nyquist sampling criterion. The discretization of the y-axis of the sinogram is the same with the discretization of the x- and y-axes on the image: N positions from -FOV/2 to FOV/2. However, it is always possible to do undersampling or oversampling. In other words, one can use broader or finer steps to define the sinogram.

**Fig. 13** shows a 2D-sinogram (sinogram domain) with a superimposed trajectory T. Each dot of the trajectory T represents one trajectory section linked with an individual system matrix. The sinogram comprises columns each column referring to an FFL-angle $\theta$ and rows each row referring to FFL-offsets r. In a 3D-sinogram, a third dimension z is added referring to the target positions of the source perpendicular to the focus-field directions (not shown). The cells of a sinogram contain reconstructed image data (not shown) corresponding to trajectory sections with the according FFL-angle, FFL-offset and target position. The data-subsets within one column of the sinogram are supposed to originate from calibration measurements with the same FFL-angle (wherein "same" may comprise an interval depending on sinogram resolution).

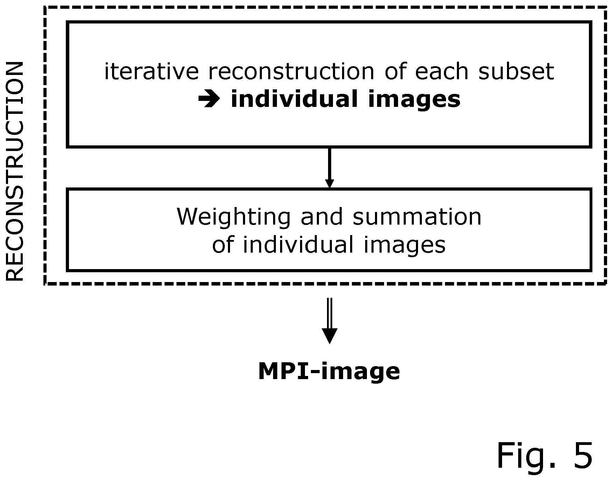

**Fig. 5-9** show different reconstruction variants.

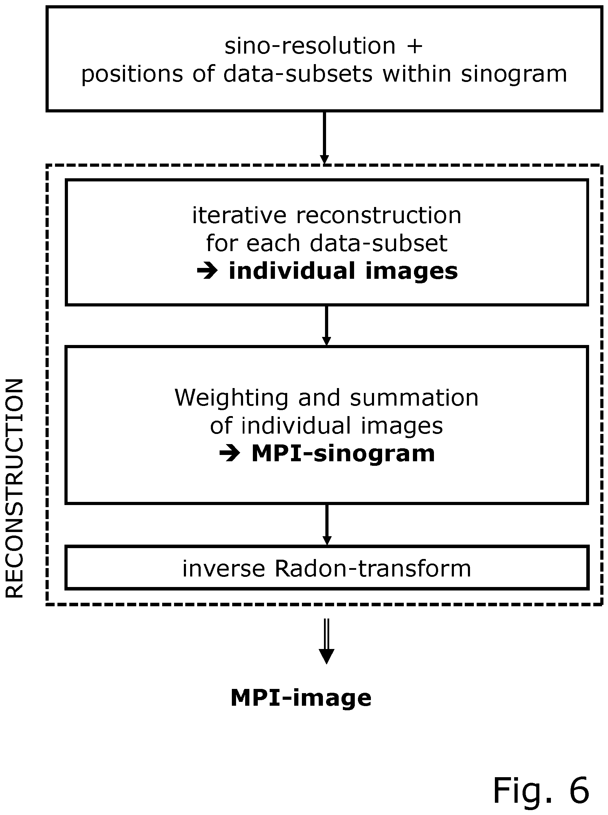

**Fig. 5 and Fig. 6** show single section based variants, in which iterative reconstruction is performed for each data-subset, thereby generating individual images (one for each data-subset). The individual images are weighted and summed up. In case an FFP has been used during MPI-data acquisition, the summation of all individual images results in the MPI-image (Fig. 5):

$$\text{MPI-image}(x, y, z) = \sum_{c=1}^{C} x_c(x - r_{x_c}, y - r_{y_c}, z)$$

where $r_{x_c}$ and $r_{y_c}$ are the FFR offsets in the x- and y-directions of the respective data-subsets, respectively. In case an FFL has been used during MPI-data acquisition, the summation of all individual images results in an MPI-sinogram (Fig. 6).

$$\text{MPI-sinogram}(r, \theta, z) = \sum_{\substack{c=1 \\ \theta_c \sim \theta}}^{C} x_c(r - r_c, z)$$

where $r_c$ is the FFR offset of the respective data-subsets, and $\theta_c \sim \theta$ is 1 or 0, representing that FFL-angle $\theta_c$ of the respective trajectory-section is assigned or not assigned to the sinogram column $x_\theta$, respectively. An MPI-sinogram represents the Radon transform of the MPI-image. I.e. each column of the MPI-sinogram corresponds to projection of the MPI-image at the FFL-angle associated with that specific column. An inverse Randon transform is applied to the MPI-sinogram in order to obtain the MPI-image:

$$\text{MPI-image} = \mathcal{R}^{-1}\{\text{MPI-sinogram}\}$$

where $\mathcal{R}^{-1}$ is the inverse Radon transform operator. Optionally, the inverse Radon transform operation can also be performed iteratively using a discrete inverse Radon transform matrix.

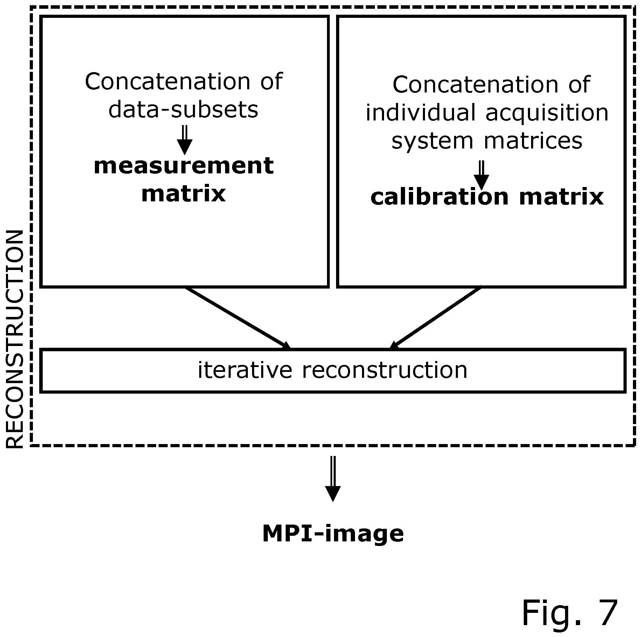

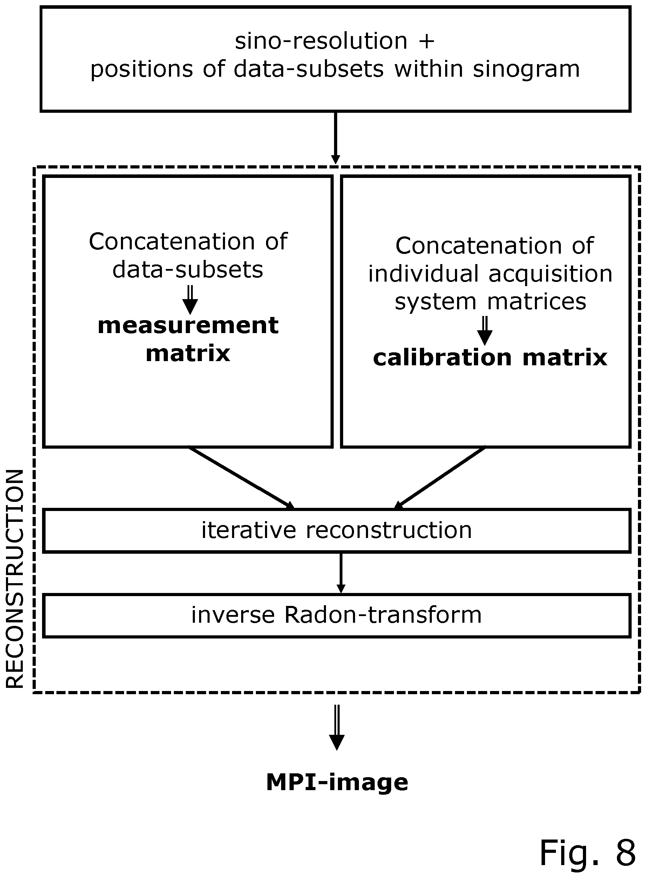

**Fig. 7 and Fig. 8** show one-shot variants, in which all data-subsets are shifted by associated FFR-offsets of trajectory section *c* and concatenated resulting in the measurement vector and all selected individual acquisition system matrices are concatenated resulting the calibration matrix $\boldsymbol{A_{traj}}$ for FFR is FFP:

$$\boldsymbol{A_{traj}}(x, y, z) = \begin{bmatrix} A_1(f, x - r_{x_1}, y - r_{y_1}, z) \\ A_2(f, x - r_{x_2}, y - r_{y_2}, z) \\ \vdots \\ A_C(f, x - r_{x_C}, y - r_{y_C}, z) \end{bmatrix},$$

and for FFR is FFL:

$$\boldsymbol{A_{traj}}(r, \theta, z) = \begin{bmatrix} A_1(f, r - r_1, z) \cdot [\theta_1 \sim \theta] \\ A_2(f, r - r_2, z) \cdot [\theta_2 \sim \theta] \\ \vdots \\ A_C(f, r - r_C, z) \cdot [\theta_C \sim \theta] \end{bmatrix}.$$

The MPI-image is achieved by only one iterative reconstruction. In case an FFP has been used during MPI-data acquisition, the reconstruction can be directly carried out by applying the optimization algorithm to the measurement vector (Fig. 7). In case an FFL has been used during MPI-data acquisition, the iterative reconstruction results in a sinogram. An inverse Randon transform is applied to the sinogram in order to obtain the MPI-image (Fig. 8). Optionally, the inverse Radon transform operation can also be performed iteratively using a discrete inverse Radon transform matrix.

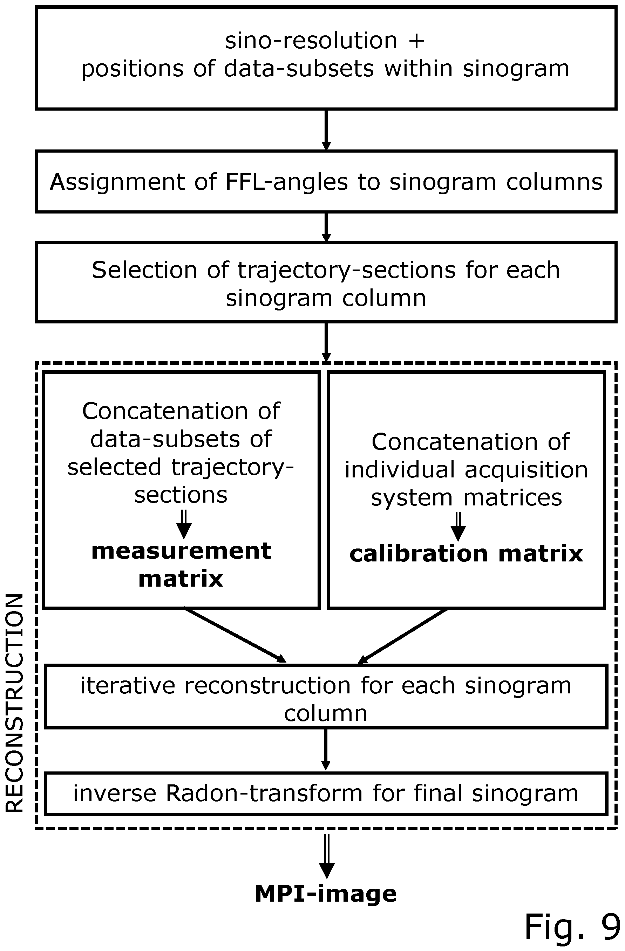

**Fig. 9** shows a column based FFL-variant, in which each FFL-angle of the trajectory is assigned to a sinogram column. All data-subsets acquired during trajectory-sections having the same FFL-angle are concatenated resulting in the measurement vector. All individual acquisition system matrices associated with the data subsets having the same FFL-angle are shifted by the associated FFR-offset of trajectory section c and concatenated resulting in the calibration matrix. The iterative reconstructions result in columns of the sinogram. An inverse Randon transform is applied to the sinogram in order to obtain the MPI-image. Optionally, the inverse Radon transform operation can also be performed iteratively using a discrete inverse Radon transform matrix.

List of reference signs

CV calibration volume

ROI region of interest

OS overscanning region

T trajectory (MPI sequence)

FFL field free line

List of cited documents

| [Kaethner] | Kaethner et al., "Axially Elongated Field-Free Point Data Acquisition in Magnetic Particle Imaging," IEEE Transactions on Medical Imaging, vol. 34, no. 2, pp. 381-387, Feb. 2015, doi: 10.1109/TMI.2014.2357077. |
| [Von Gladiss] | Von Gladiss, A., et al. "Hybrid system calibration for multidimensional magnetic particle imaging." Physics in Medicine & Biology 62.9 (2017): 3392 |
| [Halkola] | Halkola et al. "System Calibration Unit for Magnetic Particle Imaging: Focus Field Based System Function" Buzug, T., Borgert, J. (eds) Magnetic Particle Imaging. Springer Proceedings in Physics, vol 140. Springer, Berlin, Heidelberg. doi: 10.1007/978-3-642-24133-8_5 |
| [CN115797493] | CN115797493 |
| [US2015221103] | US 2015 221 103. |
| [US20170020407] | US20170020407A1 |
| [US009651637] | US009651637B2 |

**Claims**

1. Method for generating a multi-dimensional system matrix A for MPI-measurements, the method comprising:

 • prior to calibration measurements:

  ◦ determine

   - target positions for a calibration source,
   - calibration voxels of a calibration volume (CV) from which calibration data is to be acquired, the calibration volume (CV) comprising a region of interest (ROI)
   - parameters, wherein the parameters include FFR velocities of a field free region FFR of a magnetic selection field,
   - FFR-offsets r, by which the field free region FFR is to be shifted within the calibration volume (CV),
   - acquisition window, which is the duration of one calibration measurement to be performed,

  ◦ discretize parameters, thereby generating intervals of discretized parameter values for each parameter,

 • perform calibration measurements by applying at least one drive field cycle of a drive field in each calibration measurement and acquire the calibration data from the calibration voxels,
 wherein during each calibration measurement, data is acquired for a specific parameter value combination,
 • determine individual acquisition system matrices $A_c$, wherein each individual acquisition system matrix $A_c$ combines calibration data derived from different calibration measurements that are assigned to the same parameter value combination but different FFR-offsets r, and
• determine the multi-dimensional system matrix *A* by combining individual acquisition system matrices $A_c$ of different parameter value combinations.

2. Method according to claim 1, **characterized in that** for setting a specific FFR-offset r, the field free region is shifted in focus-field direction by applying according focus fields and/or in drive field direction by mechanically shifting the calibration source.

3. Method according to one of the preceding claims, **characterized in that** for each FFR-offset r, calibration data is acquired from each calibration voxel for different parameter value combinations and/or for each parameter value combination, calibration data is acquired from each calibration voxel.

4. Method according to one of the preceding claims, **characterized in that** during each calibration measurement, calibration data is acquired from one specific calibration voxel only.

5. Method according to one of the claims 1 to 4, **characterized in that**:

   i. the calibration source is positioned within the region of interest (ROI) at a first target position,
   ii. calibration measurements with the calibration source being positioned at a first target position are performed, wherein for each calibration measurement the field free region is offset to a specific calibration voxel, and wherein calibration measurements are performed for all predefined parameter value combinations until all FFR-offsets r have been set.
   iii. the calibration source is positioned within the region of interest (ROI) at a further target position that is shifted relative to the previous target position within the region of interest (ROI) in at least one direction, preferably along a drive field direction, and
   iv. calibration measurements with the calibration source being positioned at the further target position are performed,
   v. steps iii - iv are repeated until measurements for all target positions of the calibration source are performed.

6. Method according to claim 5 , **characterized in that** background measurements are performed, preferably prior to positioning the calibration source at the target positions in step i and step iii, without the calibration source being in the calibration volume (CV) and/or with the calibration source being in a magnetically saturated state.

7. Method according to any of the preceding claims, **characterized in that** the discretization is performed such that the parameter values are uniformly or non-uniformly spaced.

8. Method according to claim 1 to 7, **characterized in**

   **that** parameter value combinations are determined from a predetermined MPI-sequence, and
   **that** calibration measurements with all parameter value combinations specific for the MPI-sequence are performed.

9. Method according to one of the claims 1 to 8, **characterized in that** the field free region is a field free line (FFL), and that the parameters comprise FFL-angle θ, FFL-angular-velocity, FFL-translational-velocity.

10. Method according to one of the claims 1 to 8, **characterized in that** the field free region is a field free point, and that the parameters comprise FFP-velocities in any spatial direction.

11. Method according to any of the preceding claims **characterized in that** in order to move the calibration source between the target positions, the calibration source is moved relative to the region of interest (ROI) exclusively in the drive field direction.

12. Method according to any of the preceding claims, **characterized in that** the calibration volume (CV) consists of n voxels, wherein the number m of calibration voxels is smaller than the number n of voxels of the calibration volume (CV), and that calibration data for voxels not being calibration voxels is interpolated and/or extrapolated from the already acquired calibration data of the calibration voxels.

13. Method for generating an MPI-image from MPI-data which have been acquired by performing an MPI sequence (T), the method using a multi-dimensional system matrix A generated according to one of the claims 1 to 12, the method comprising

- dividing the MPI-sequence into trajectory-sections, whereby the duration of each trajectory-section corresponds to the duration of the acquisition window, each trajectory-section being associated with an actual parameter value combination, wherein during each trajectory-section a data-subset of the MPI-data is acquired,
- for each trajectory-section: picking the best matching individual acquisition system matrix $A_c$ from the multi-dimensional system matrix $A$, wherein the best matching individual acquisition system matrix $A_c$ is the one with the parameter value combination that best matches the actual parameter value combination of the respective trajectory-section, where $c^{+\mathbb{Z}} \in [1, C]$ is the trajectory-section number and $C$ is the total number of trajectory-sections,
- determine an image-resolution of the MPI-image to be generated, the image-resolution comprising voxel sizes and FOV size,
- choose an optimization algorithm,
- reconstruct the MPI-image by applying the chosen optimization algorithm to the MPI-data using the picked best matching individual acquisition system matrices $A_c$.

14. Method according to claim 13, **characterized in**

   **that** the field free region is a field free point, and
   **that** the MPI-image is reconstructed by performing the following steps:

   - for each data-subset, perform iterative reconstruction, wherein each reconstruction results in an individual image $x_c$
   - weight and sum up all individual images $x_c$, resulting in the MPI-image.

15. Method according to claim 13, **characterized in**

   **that** the field free region is a field free point, and
   **that** the MPI-image is reconstructed by performing the following steps:

   - concatenate all data-subsets, resulting in a measurement vector $b_{traj}$
   - concatenate all individual acquisition system matrices $A_c$ with the same order used for forming $b_{traj}$, resulting in a calibration matrix $A_{traj}$,
   - to obtain the MPI-image: perform iterative reconstruction.

16. Method according to claim 13, **characterized in**

   **that** the field free region is a field free line (FFL), and
   **that** prior to reconstruction the following steps are performed:

   - for the given image resolution, determine a corresponding sino-resolution of a sinogram to be generated, the sino-resolution comprising discretized FFL-angles θ and FFL-offsets r,
   - determine positions of the data-subsets within the sinogram.

17. Method according to claim 16, **characterized in**
   **that** the MPI-image is reconstructed by performing the following steps:

   - for each data-subset, perform iterative reconstruction, wherein each reconstruction results in an individual image $x_c$
   - weight and sum up all individual images $x_c$, resulting in the sinogram, and
   - apply an inverse Radon transform to the sinogram resulting in the MPI-image.

18. Method according to claim 16, **characterized in**
   **that** the MPI-image is reconstructed by performing the following steps:

   - assign each FFL-angle θ of the trajectory-sections to one column of the sinogram,

- for each sinogram column: select trajectory-sections, whose FFL-angle $\theta$ was assigned to the respective sinogram column,
- concatenate all data-subsets acquired during the selected trajectory-sections, resulting in a measurement vector $b_\theta$,
- concatenate all individual acquisition system matrices $A_c$ with the same order used for forming measurement vector $b_\theta$, resulting in a calibration matrix $A_\theta$,
- perform iterative reconstruction for each discretized FFL-angle $\theta$, resulting in columns $x_\theta$, of a reconstructed final sinogram,
- to obtain the MPI-image: apply an inverse Radon transform to the reconstructed final sinogram.

| determine | -target positions |
| | -calibration voxels |
| | -parameters |
| | -FFR-offsets |
| | -acquisition window |

$\downarrow$

| discretization of parameters |

$\downarrow$

| calibration measurements |

$\downarrow$

| individual acquisition system matrices $A_C$ |

$\downarrow$

| Combination of individual acquisition system matrices |

$\Downarrow$

**multidimensional system matrix _A_**

Fig. 1

Fig. 2

background measurements

set first target position

set first FFR-offset

calibration measurement
(vary parameter value combination)

further FFR-offset?

set further FFR-offset

further target position?

set further target position

background measurements

end of data acquisition

yes

no

yes

no

Fig. 3

EP 4 749 313 A1

**MPI-image**

Fig. 4

RECONSTRUCTION

iterative reconstruction of each subset
→ **individual images**

Weighting and summation
of individual images

**MPI-image**

Fig. 5

sino-resolution +
positions of data-subsets within sinogram

RECONSTRUCTION

iterative reconstruction
for each data-subset
→ **individual images**

Weighting and summation
of individual images
→ **MPI-sinogram**

inverse Radon-transform

**MPI-image**

Fig. 6

Fig. 7

Fig. 8

**RECONSTRUCTION**

sino-resolution +
positions of data-subsets within sinogram

↓

Assignment of FFL-angles to sinogram columns

↓

Selection of trajectory-sections for each
sinogram column

↓

Concatenation of
data-subsets of
selected trajectory-
sections

⇓

**measurement
matrix**

Concatenation of
individual acquisition
system matrices

⇓

**calibration matrix**

iterative reconstruction for each sinogram
column

↓

inverse Radon-transform for final sinogram

⇓

**MPI-image**

Fig. 9

Fig. 10

Fig. 11

Fig. 12

Fig. 13

Europäisches
Patentamt

European
Patent Office

Office européen
des brevets

# EUROPEAN SEARCH REPORT

**Application Number**

EP 24 21 4299

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TOP CAN BARIS ET AL: "Tomographic Field Free Line Magnetic Particle Imaging With an Open-Sided Scanner Configuration", IEEE TRANSACTIONS ON MEDICAL IMAGING, IEEE, USA, vol. 39, no. 12, 3 August 2020 (2020-08-03), pages 4164-4173, XP011822934, ISSN: 0278-0062, DOI: 10.1109/TMI.2020.3014197 [retrieved on 2020-11-30] | 1-15 | INV. G01R33/12 A61B5/0515 |
| A | * sections B, C; figures 1, 2, 6 * | 16-18 | |
| | ----- | | |
| Y | US 2019/287277 A1 (FRANKE JOCHEN [DE] ET AL) 19 September 2019 (2019-09-19) | 1-15 | |
| A | * paragraphs [0002], [0006], [0007], [0066] - [0069]; claim 1; figures 1-4 * | 16-18 | |
| | ----- | | |
| Y | HE JIE ET AL: "Sequential Scan-Based Single-Dimension Multi-Voxel System Matrix Calibration for Open-Sided Magnetic Particle Imaging", IEEE TRANSACTIONS ON MEDICAL IMAGING, IEEE, USA, vol. 43, no. 11, 22 May 2024 (2024-05-22), pages 3856-3868, XP011987896, ISSN: 0278-0062, DOI: 10.1109/TMI.2024.3404602 [retrieved on 2024-05-23] | 1-15 | TECHNICAL FIELDS SEARCHED (IPC) G01R A61B |
| A | * sections I, II; figures 1-4 * | 16-18 | |
| | ----- | | |
| Y | US 2019/285710 A1 (FRANKE JOCHEN [DE] ET AL) 19 September 2019 (2019-09-19) | 1-15 | |
| A | * paragraphs [0047], [0048]; figures 4, 5 * | 16-18 | |
| | ----- | | |
| | -/-- | | |

The present search report has been drawn up for all claims

| Place of search | Date of completion of the search | Examiner |
|---|---|---|
| Munich | 10 April 2025 | Philipp, Peter |

EPO FORM 1503 03.82 (P04C01)

page 1 of 2

Europäisches
Patentamt

European
Patent Office

Office européen
des brevets

## EUROPEAN SEARCH REPORT

Application Number

EP 24 21 4299

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | US 2017/020407 A1 (WEBER ALEXANDER [DE]) 26 January 2017 (2017-01-26) * paragraphs [0008], [0009], [0021], [0045] - [0048]; figures 1-5 * ----- | 10,12 | |
| | | | **TECHNICAL FIELDS SEARCHED (IPC)** |

The present search report has been drawn up for all claims

| Place of search | Date of completion of the search | Examiner |
|---|---|---|
| Munich | 10 April 2025 | Philipp, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 4299

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2025

| Patent document cited in search report | | Publication date | Patent family member(s) | | Publication date |
|---|---|---|---|---|---|
| US 2019287277 | A1 | 19-09-2019 | DE 102018203786 A1 | | 19-09-2019 |
| | | | US 2019287277 A1 | | 19-09-2019 |
| US 2019285710 | A1 | 19-09-2019 | CN 110269614 A | | 24-09-2019 |
| | | | DE 102018203783 B3 | | 21-02-2019 |
| | | | US 2019285710 A1 | | 19-09-2019 |
| US 2017020407 | A1 | 26-01-2017 | CN 106419914 A | | 22-02-2017 |
| | | | DE 102015214071 B3 | | 22-09-2016 |
| | | | EP 3120765 A1 | | 25-01-2017 |
| | | | US 2017020407 A1 | | 26-01-2017 |

EPO FORM P0459

For more details about this annex : see Official Journal of the European Patent Office, No. 12/82

REFERENCES CITED IN THE DESCRIPTION

**Patent documents cited in the description**

- US 2015221103 A
- US 20170020407 A
- CN 115797493
- US 009651637 B
- US 20170020407 A1
- US 009651637 B2

**Non-patent literature cited in the description**

- **KAETHNER et al.** Axially Elongated Field-Free Point Data Acquisition in Magnetic Particle Imaging. *IEEE Transactions on Medical Imaging*, February 2015, vol. 34 (2), 381-387
- **VON GLADISS, A. et al.** Hybrid system calibration for multidimensional magnetic particle imaging. *Physics in Medicine & Biology*, 2017, vol. 62 (9), 3392
- System Calibration Unit for Magnetic Particle Imaging: Focus Field Based System Function. **HALKOLA et al.** Magnetic Particle Imaging. Springer Proceedings in Physics. Springer, vol. 140